# EUROPEAN PATENT APPLICATION

(11) **EP 2 128 227 A1**
(43) Date of publication of application: **02.12.2009**
(21) Application number: 08075503.6
(22) Date of filing: 19.05.2008
(51) Int. Cl.: C10L 1/185, C10L 10/12, C07D 307/12, C07D 307/42

(54) **Monosubstituted furan derivatives via decarboxylation and use thereof as (aviation) fuel**

(71) Applicant: Furanix Technologies B.V, 1014 BV Amsterdam (NL)
(72) Inventor: Gruter, Gerardus Johannes Maria, 2106 BA Heemstede (NL); de Jong, Edserd, 6703 BR Wageningen (NL)
(74) Representative: Kortekaas, Marcel C.J.A.

(57) **Abstract**

The invention provides a process for preparing a monosubstituted furan derivative, for use as fuel component, by reacting a 5-(alkoxymethyl)furfural under decarbonylation conditions. Moreover, the invention also provides a process for preparing a monosubstituted tetrahydrofuran derivative, for use as fuel component, wherein the monosubstituted furan derivative is converted under hydrogenation conditions to the tetrahydrofuran analog. The invention provides further novel and useful monosubstituted furan derivatives and monosubstituted tetrahydrofuran derivatives. Hence these are also claimed as an invention. Moreover, the monosubstituted furan derivatives and monosubstituted tetrahydrofuran derivatives disclosed herein have never been used as fuel component, and therefore the use of these derivatives has also been claimed as an invention

## Description

### Technical Field:

The present invention relates to a method for the preparation of monosubstituted furan derivatives from HMF-based precursors via decarbonylation of the corresponding furfural precursors and use thereof as (aviation) fuel component, i.e., as fuel by itself or as fuel additive. More in particular, the invention relates to derivatives obtained from 5-(alkoxymaethyl)furfural (RMF) and to their application as a fuel component, more in particular as an aviation fuel component. The expression "fuel component" as used hereinafter includes both the use as fuel or as component in a blend of fuels (e.g., in an amount from 1 to 100 vol%).

### Background Art:

Gasoline, diesel and kerosene are the most commonly used liquid transportation fuels. It is known that aviation fuel is a specialized type of petroleum-based fuel used to power aircraft. It is generally of a higher quality than fuels used in less critical applications such as heating or road transport, and often contains additives to reduce the risk of icing or explosion due to high temperatures, amongst other properties. Most aviation fuels available for aircraft are kinds of petroleum spirit used in engines with spark plugs i.e. piston engines and Wankel rotaries or fuel for jet turbine engines which is also used in diesel aircraft engines.

Aviation fuels consist of blends of over a thousand chemicals, primarily hydrocarbons (paraffins, olefins, naphthenes, and aromatics) as well as additives such as antioxidants and metal deactivators, and impurities. Principal components include n-octane and isooctane. Like other fuels, blends of Aviation fuel used in piston engined aircraft are often described by their octane rating. The net energy content for present day aviation fuels depends on their composition. Some typical values are: Avgas, 43.7 MJ/kg or 31.0 MJ/L; Wide-cut jet fuel, 43.5 MJ/kg or 33.2 MJ/L, and Kerosene type jet fuel, 43.8 MJ/kg or 35.1 MJ/L.

Outside former communist areas, there are currently two main grades of turbine fuel in use in civil commercial aviation: Jet A-1 and Jet A, both are kerosene type fuels. There is another grade of jet fuel, Jet B which is a wide cut kerosene (a blend of gasoline and kerosene) but it is rarely used except in very cold climates. Jet A-1 is a kerosene grade of fuel suitable for most turbine engined aircraft. It is produced to a stringent internationally agreed standard, has a flash point above 38°C (100°F) and a freeze point maximum of - 47°C. Jet A is a similar kerosene type of fuel as Jet A-1 but with a higher freeze point maximum (-40°C). Jet B is a distillate covering the naphtha and kerosene fractions. It can be used as an alternative to Jet A-1 where its better cold weather performance is important.

JP-4 is the military equivalent of Jet B with the addition of corrosion inhibitor and anti-icing additives. JP-5 is a high flash point kerosene. Finally, JP-8 is the military equivalent of Jet A-1 with the addition of corrosion inhibitor and anti-icing additives.

As mentioned, aviation fuel typically contains additives added to the fuel in very small quantities, usually measurable only in parts per million, to provide special or improved qualities. The quantity to be added and approval for its use in various grades of fuel is strictly controlled by the appropriate specifications. A few additives in common use are as follows:
1. Anti-knock additives reduce the tendency of gasoline to detonate. Tetra-ethyl lead (TEL) is the only approved anti-knock additive for aviation use and has been used in motor and aviation gasolines since the early 1930s.
2. Anti-oxidants prevent the formation of gum deposits on fuel system components caused by oxidation of the fuel in storage and also inhibit the formation of peroxide compounds in certain jet fuels.
3. Static dissipater additives reduce the hazardous effects of static electricity generated by movement of fuel through modern high flow-rate fuel transfer systems. Static dissipater additives do not reduce the need for 'bonding' to ensure electrical continuity between metal components (e.g. aircraft and fuelling equipment) nor do they influence hazards from lightning strikes.
4. Corrosion inhibitors protect ferrous metals in fuel handling systems, such as pipelines and fuel storage tanks, from corrosion. Some corrosion inhibitors also improve the lubricating properties (lubricity) of certain jet fuels.
5. Fuel System Icing Inhibitors (Anti-icing additives) reduce the freezing point of water precipitated from jet fuels due to cooling at high altitudes and prevent the formation of ice crystals which restrict the flow of fuel to the engine. This type of additive does not affect the freezing point of the fuel itself. Anti-icing additives can also provide some protection against microbiological growth in jet fuel.
6. Metal de-activators suppress the catalytic effect which some metals, particularly copper, have on fuel oxidation.
7. Biocide additives are sometimes used to combat microbiological growths in jet fuel, often by direct addition to aircraft tanks; as indicated above some anti-icing additives appear to possess biocidal properties.
8. Thermal Stability Improver additives are sometimes used in military JP-8 fuel, to produce a grade referred to as JP-8+100, to inhibit deposit formation in the high temperature areas of the aircraft fuel system.

As mentioned above, alcohol, alcohol mixtures and other alternative fuels may have been used experimentally but are not generally available.

Although alternative aviation fuels are not general available, there are nonetheless many patents and scientific articles on (aviation) fuels containing for instance furan derivatives such as furfural, furfuryl alcohol, methylfuran, and dimethylfuran. The most important examples are summarized below.

Already in US2599338, fuel compositions are described containing tetrahydrofurfuryl alcohol. The presence of this component reduced the loss in speed due to icing both in motor fuel and in an aviation fuel.

Recently, in WO2008013922, fully renewable engine fuels were described that are said to be derived completely from biomass sources. The fully renewable engine fuel is comprised of one or more low carbon number esters derived from ethanol, one or more pentosan-derivable furans, one or more aromatic hydrocarbons, one or more C4-C10 straight chain alkanes derivable from polysaccharides, and one or more bio-oils. In addition, the fuel may contain triethanolamine. Such a lower octane renewable fuel may be utilized, for example, in automobile fuel, 100 LL aviation fuel applications, and turbine engine applications. These ethanol-based, fully renewable fuels may be formulated to have a wide range of octane values and energy, and may effectively be used to replace 100LL aviation fuel (known as "aviation gasoline" or "AvGas"), as well as high octane, rocket, diesel, and turbine engine fuels.

As discussed in this reference, the inherent energy contained within gasoline is directly related to mileage, not to octane number. Automobile gasoline has no energy specification, hence no mileage specification. In contrast, aviation fuels, a common example being 100 LL (100 octane low lead), do have an energy content specification. This translates to aircraft range and to specific fuel consumption. Aircraft cannot compromise range. For this reason, energy content is equally important as MON values. It should be realized, however, that the aforementioned ethanol-based fuels are produced through fermentation of food crops and such and therefore compete with the production of food products. The generation of biofuel from food crops is widely discussed, given the pressure it will put on land, prices of food and the hunger problem.

Furan derivatives have been used for various purposes in fuel compositions. In e.g., US20060180786A tetrahydrofurfuryl acetate and other derivatives have been described as pour point depressant for jet fuel/diesel fuel compositions. More specifically, this reference provides a method for lowering the pour point of biodiesel, said method comprising preventing or reducing crystallization of fatty acids, esters or alkaline salts of fatty acids in the biodiesel product by adding to said biodiesel an effective crystallization reducing or preventing amount of a composition, said composition comprising a.o. tetrahydrofurfuryl acetate. Indeed, as antifreeze component, furfuryl alcohol has already been described in US2229215.

Furan derivatives have been suggested also for use in hypergolic propellants. A hypergolic propellant is either of the two rocket propellants used in a hypergolic rocket engine, which spontaneously ignite when they come into contact. The two propellants are usually termed the "fuel" and the "oxidizer". Although hypergolic propellants tend to be difficult to handle, a hypergolic engine is easy to control and very reliable. In common usage, the terms "hypergol" or "hypergolic propellant" are often used to mean the most common such propellant combination, hydrazine plus nitrogen tetroxide, or their relatives. Examples of furan derivatives in hypergolic propellants may be found in US4316359, US2994191, US2993334, and US2874535.

US4339245 describes a gasoline composition containing at least one antiknock compound selected from the group consisting of furfuryl acetate, ethyl furfurylacrylate, methyl furoate, and ethyl furoate. This case is not specific to aviation fuels. Similar cases on antiknock components, cetane improvers, or component to improve the octane rating may be found in US3359087, US3021204, EP1321504, WO2005087901, US5925152, US5697987 (which describes 2-methyltetrahydrofuran as an anti-knock component for spark ignition motor fuel composition at levels between 15-55%), US4522630, US2321311 (which discloses a motor fuel composition comprising a mixture of gasoline hydrocarbons adapted as a base fuel for spark-ignition engines improved in antiknock value by an addition of a substantial amount of at least 1% by volume of a heterocyclic compound containing a furane nucleus and selected from the group consisting of furane, alkyl furanes, furfuryl alcohols, furfuryl amines, and the saturated derivatives thereof, said heterocyclic compound boiling in the range of said base fuel), US2003110684 and many other references.

From US2003154649 a method is known for reducing the vapor pressure of a C3 to C12 hydrocarbon-based motor fuel composition for a conventional spark ignition internal combustion engine comprising combining: (a) a hydrocarbon component comprising C3 to C12 hydrocarbon fractions; (b) an ethanol component comprising fuel grade ethanol, said ethanol component comprising 0.1 % to 20% of the composition by volume; (c) an oxygen-containing heterocyclic compound having 5 to 8 carbon atoms selected from the group consisting of tetrahydrofurfuryl alcohol, tetrahydrofurfuryl acetate, dimethyltetrahydrofuran, tetramethyltetrahydrofuran.

US2002053161 discloses the use of oxygenated compounds derived from tetrahydrofurfuryl as additives or formulation bases of gas-oils and leading to a significant lowering of particle emissions. The oxygenated compound has the formula THF-C-X-R in which -X- is chosen from -O- and -O-(CH2-O)n (n=1-20) and R is chosen from alkyl groups containing from 1 to 30 carbon atoms; and groups containing a tetrahydrofurfuryl unit (isopropyl, isobutyl, tertbutyl and tertamyl groups).

Unfortunately, such derivatives are relatively expensive to make, they are relative unstable and/or their physical/chemical properties (energy density, melting point, boiling points, flash points, viscosity) are suboptimal.

Strongly increasing fossil fuel prices, security of supply issues and growing concerns about greenhouse gas emissions have intensified the search for and use of Bioenergy applications (solar, wind, biomass, geothermal etc). An important area is the transportation sector but unfortunately on the short term only limited renewable energy solutions are available. From an environmental perspective hydrogen would be a very attractive fuel. Unfortunately, hydrogen is a very inconvenient energy storage medium. Thus, the density of hydrogen at room temperature and pressure is 0.00009 kg/m3. The density at 700 bar amounts to 57.5 kg/m3, with an energy density of Qv=120 MJ/kg. The density of gasoline is 740 kg/m3, Qv=44 MJ/kg. Although per kg, hydrogen has a 120/44 = 2.7 x higher energy density than gasoline, because of the low mass per volume, even at 700 bars hydrogen has only (2.7 * (57.5/740)) 21% of the energy content of gasoline. In addition, compression to 700 bar requires 20% of fuel energy. Hydrogen can also be stored as a liquid in a cryogenic tank. However, when stored at -253°C the density is still only 70 kg/m3. This liquefaction requires 40% of fuel energy. The required fuel tanks (for both pressurization and liquefaction) are heavy and expensive. A cryogenic tank to store 8 kg fuel weighs 120 kg - high pressure tanks are significantly heavier! A major disadvantage of liquefaction is that half the fuel boils off in 9 days!

A further solution has been to store hydrogen in mineral or organic materials. Metallocarboranes have been able to reversibly store 50 kg/m3 hydrogen (NREL annual hydrogen report 2007). These materials are however prohibitively expensive. So far the strategies for hydrogen storage via reaction or complex formation with "host" materials required release of hydrogen upon use.

Another fuel considered attractive from an environmental perspective is biofuel. Therefore, interest in biofuels as a renewable source for all different transportation fuels has strongly increased. Biofuel can be broadly defined as solid, liquid, or gas fuel consisting of, or derived from recently dead biological material, most commonly plants. This distinguishes it from fossil fuel, which is derived from long dead biological material. Biofuels are used globally. The most common use for biofuels is automotive transport. The use of renewable biofuels in lieu of fossil fuels is said to reduce greenhouse gas emissions and increase energy security.

From WO2007104514 a method is known for the manufacture of 5-(alkoxymethyl)furfural derivatives by reacting a fructose and/or glucose-containing starting material with an alcohol in the presence of a catalytic or sub-stoichiometric amount of heterogeneous (solid) acid catalyst. The catalysts may be employed in a continuous flow, fixed bed, or catalytic distillation reactor. The ethers can be applied as a fuel or fuel additive. For fuel and aviation fuel in particular, products of even higher energy density and even better (low temperature) blend properties than 5-(alkoxymethyl)furfural are required. Ideally, these improved products should be based on biomass or biomass products. On the other hand, the preparation of these (aviation) biofuels should be economically feasible and energetically acceptable. Interestingly, the current inventors have found a new class of compounds that may be used as fuel and/or as fuel additive. These compounds may be produced by proven technologies, using starting materials based on biomass or biomass products.

### Disclosure of the Invention:

Accordingly, the invention provides a process for preparing a monosubstituted furan derivative, for use as fuel component, by reacting a 5-(alkoxymethyl)furfural under decarbonylation conditions.

Moreover, the invention also provides a process for preparing a monosubstituted tetrahydrofuran derivative, for use as fuel component, wherein the monosubstituted furan derivative is converted under hydrogenation conditions to the tetrahydrofuran analog. The invention provides further novel and useful monosubstituted furan derivatives and monosubstituted tetrahydrofuran derivatives. Hence these are also claimed as an invention. Moreover, the monosubstituted furan derivatives and monosubstituted tetrahydrofuran derivatives disclosed herein have never been used as fuel component, and therefore the use of these derivatives has also been claimed as an invention.

### Mode(s) for carrying out the Invention:

The synthesis of a 5-(alkoxymethyl)furfural is already described in the international application mentioned above, WO2007104514, herein included by reference. This application provides a very interesting route to such furfural derivatives by conversion of C6 sugars. This route is of particular interest as it competes positively with the approach involving the synthesis of these components from furfural via etherification of furfuryl alcohol.

On the other hand, the melting point, the energy density and the blending properties of e.g. 5-(ethoxymethyl)furfural (EMF) leaves room for improvement. This is of particular importance in aviation fuels, which require amongst others a flash point above 38 °C and a freezing point maximum of -47°C (Jet A-1 standards).

For this invention, any 5-(alkoxymethyl)furfural may be used, including mixtures of different 5-(alkoxymethyl)furfurals as well as mixtures of a 5-(alkoxylmethyl)furfural and other furfural derivatives such as 5-(hydroxymethyl)furfural. Thus, the alkoxy group may have from 1 (methoxy) to 20 carbon atoms, it may be linear or branched, and it may even contain O and N elements both in the chain (e.g. on the beta position or further on in the chain) and as a side-group. For sake of convenience, such groups are still referred to as alkoxy groups. Indeed, the alkoxy group may contain various inert substituents or even carbon-carbon double bonds, although this is not preferred. Preferably the alkoxy group contains from 1 to 12 carbon atoms, more preferably from 1 to 6 carbon atoms, more preferably 1 to 4 carbon atoms.

In itself, the catalytic decarbonylation of furfural into furan has been described in patents before.

Systems using different kinds of catalysts are described in US3007941, US2776981, US4764627. For instance, in the latter a process is disclosed for producing furan from furfural using a zeolite catalyst.

Also in the open literature catalytic systems for decarbonylation have been described before. Some examples thereof include the articles by Jung et al (Bulletin de la Société Chimique de France, 459-464, 1986; Biomass 16:63-76, 1988; and Biomass 16:89-96, 1988); by H.E. Exchinazi (Bull. Soc. Chim. 967-969, 1952).

Moreover, from US2003055271 a process is known for the preparation of furan comprising converting 2,5-diformylfuran into furan and furfural via decarbonylation in the presence of a catalyst consisting essentially of an optionally supported metal selected from Periodic Group VIII.

In US4089871 a method of producing furfuryl alcohol has been described comprising: contacting hydroxymethylfurfural with a catalyst selected from the group palladium and rhodium.

As C6 sugars such as glucose and fructose are much cheaper and much more easily available than C5 sugars such as xylose, a route to 2-(alkoxymethyl)furan from C6 sugars is more interesting than a route from C5 sugars.. However, without an economic route from C6 sugars to 5-(alkoxymethyl)furfurals, the use of these materials as a starting material for the production of 2-(alkoxymethyl)furan is less interesting. The decarbonylation of a C6 sugar derived 5-(alkoxymethyl)furfural has not been described.

Suitably, any known decarbonylation process may be used. More preferably, the decarbonylation is carried out at elevated temperatures, in the presence of a suitable homogeneous or heterogeneous catalyst. Use of a heterogeneous (e.g., solid or supported) catalyst is particularly advantageous from a process perspective (e.g., in continuous processes) and a cost perspective (less loss of catalyst). The decarbonylation catalyst for use in the present invention may therefore be selected from heterogeneous catalysts containing platinum, rhodium, ruthenium, palladium or nickel or mixture thereof (preferably in an amount of from 0.01 to 10 % by weight) and containing sodium, potassium and/or cesium metal (preferably in an amount from 0.1 to 10.0% by weight). Preferred catalysts are those that contain from 0.1 - 2.0% by weight of rhodium and/or platinum and from 0.1 to 10.0% by weight of cesium. Various supports may be used, provided they are inert and mechanically stable, such as alumina, titanium dioxide, silica, aluminium silicates, zeolites, magnesium silicate and active carbon and the like, albeit that zeolites and carbon as support are preferred. The decarbonylation catalyst may be activated by treatment with hydrogen first.

For instance, the reaction may be carried out at a hydrogen pressure of from 0.1 to 20 bar, and wherein the molar ratio of hydrogen to furfural is not less than 0.5.

The temperature at which the decarbonylation process is performed may vary, but in general it is preferred that the reaction is carried out at a temperature from 50 to 300 degrees Celsius, preferably from 80 to 250 Celsius, more preferably from 150 to 225 degrees Celsius. In general, temperatures higher than 300 are less preferred as the selectivity of the reaction deteriorates at high temperatures. Performing the reaction below the lowest temperature is also less preferable because of the slow reaction speed.

The catalyst can be added to the reaction mixture in an amount varying from 0.01 to 40 mole % drawn on the starting material preferably from 0.1 to 30 mole %, more preferably from 1 to 20 mole %.

In certain embodiments, one or more solvents or diluents may be added, for instance when they have been used as reaction solvent in the production of the 5-(alkoxymethyl)furfural starting material. Suitable solvents should be inert during the decarbonylation reaction. Suitable solvents include for instance alcohols, preferably similar to those used as the alkoxy group i.e. ethanol in case the alkoxy group is an ethoxy group.

As indicated above, the decarbonylation process can be performed in a continuous flow process. In that case, homogenous catalysts may be used and the residence time of the reactants in the flow process is between 0.1 second and 10 hours, preferably from 1 second to 5 hours, more preferably from 5 seconds to 10 minutes.

Alternatively, and according to the preferred embodiment, the continuous flow process is a fixed bed continuous flow process or a reactive (catalytic) distillation process with preferably a heterogeneous decarbonylation catalyst. To initiate or regenerate the heterogeneous catalyst or to improve performance, fresh catalyst or catalyst regenerators may be added to the feed of the fixed bed or reactive distillation continuous flow process. In a fixed bed process, the liquid hourly space velocity (LHSV) can be from 1 to 1000, preferably from 5 to 500, more preferably from 10 to 250 and most preferably from 25 to 100.

Of particular interest is the carbon monoxide generated during the decarbonylation, which may be used with water to generate hydrogen and carbon dioxide. The hydrogen can then be used in a subsequent hydrogenation process to convert the monosubstituted furan derivative prepared by decarbonylation according to the invention into the monosubstituted tetrahydrofuran analogue.

The catalytic hydrogenation of furan derivatives into their tetrahydrofuran analogs has been described in patents before. By way of example, some processes are listed hereafter.

Continuous vapor-phase processes have been disclosed in US7064222 using commercially-available catalysts, namely, a reduced copper-based catalyst consisting essentially of cupric oxide, chromium (III) oxide, manganese oxide and barium chromate and a reduced nickel-based catalyst consisting essentially of nickel, nickel (II) oxide, aluminium oxide and silica.

Hydrogenation of the furan ring of furfuryl alcohol has been described before using Ni or Cu catalysts on different kind of supports (US2838523, US3652458). In US2006128844 a method is described of preparing 2,5-bis(hydroxymethyl)tetrahydrofuran using a catalyst system comprising nickel and zirconium.

A catalytic process for the simultaneous synthesis of furfuryl alcohol and cyclohexanone by the hydrogenation of furfural and dehydrogenation of cyclohexanol respectively has been described in US7015359. The process comprising contacting a mixture of furfural and cyclohexanol with a Cu based catalyst of the formula xCu-yMgO-zCr2O3, wherein x, y and z are the amounts in terms of weight percent of Cu, MgO and Cr2O3 respectively.

From US4459419 a hydrogenation process is known, which comprising contacting 2,5-bis-(hydroxymethyl)furan with hydrogen in contact with a catalyst consisting essentially of zeolite support containing a catalytic amount of ruthenium in cationic form. Raney nickel and nickel have been claimed for the hydrogenation of furoic acid and HMF into tetrahydrofuroic acid and 2,5-bis-(hydroxymethyl)tetrahydrofuran (US3342838, US4153578)

In US2007287845 a method is provided of reducing hydroxymethylfurfural (HMF) with a catalyst containing at least one metal selected from Ni, Co, Cu, Pd, Pt, Ru, Ir, Re and Rh.

In US2003069457 a non-chrome, copper-containing catalyst, Cu--Al--O and method of preparing the same are provided. The Cu--Al--O catalyst can be employed in applications in place of Cu/Cr, or other copper based catalysts.

Similar to the main invention described above, the hydrogenation of the C6 sugar-derived 2-(alkoxymethyl)furan derivative has not been described before.

Suitably, any known hydrogenation process may be used. More preferably, the hydrogenation is carried out at elevated temperatures, in the presence of a suitable homogeneous or heterogeneous catalyst. Use of a heterogeneous (e.g., solid or supported) catalyst is particularly advantageous from a process perspective (e.g., in continuous processes) and a cost perspective (less loss of catalyst). The hydrogenation catalyst for use in the present invention may therefore be selected from heterogeneous catalysts containing base metals Ni, Cu, Co and Ca and/or Group VIII metals Pt, Pd, Rh and Ru. Most preferably, from an economics point of view the catalyst is a heterogeneous catalyst containing Ni. Most preferably, from a process point of view the catalyst is a heterogeneous catalyst containing Pd or Rh. An overview of useful catalysts is given in the Handbook of commercial catalysts by Rase (CRC press, 2000), pages 105-213. As support, any inert and mechanically stable support may be used, such as the ones mentioned hereinbefore for the decarbonylation catalyst. The most preferred supports are silica and carbon.

The temperature at which the hydrogenation process is performed may vary, but in general it is preferred that the reaction is carried out at a temperature from 50 to 400 degrees Celsius, preferably from 100 to 250, more preferably from 100 to 150 degrees Celsius. In general, temperatures higher than 300 are less preferred as the selectivity of the reaction deteriorates at high temperatures. Exceptions to this may be processes that are taking place at higher temperatures (such as 400 degrees C) in the gas-phase. Performing the reaction below the lowest temperature is also less preferable because of the slow reaction speed.

The reaction is preferably carried out a hydrogen pressure of from 1 to 100 bar, or more preferably from 5 to15 bar, where the molar ratio of hydrogen to furan derivative is not less than 5.

The catalyst can be added to the reaction mixture in an amount varying from 0.01 to 40 mole % drawn on the starting material preferably from 0.1 to 30 mole %, more preferably from 1 to 20 mole %.

A solvent may be present, e.g., if it has been used as reaction medium in the synthesis of the starting materials. Also, as mentioned before on the decarbonylation reaction, the hydrogenation process may be performed in a continuous flow process. In that case, homogenous catalysts may be used and the residence time of the reactants in the flow process is between 0.1 second and 10 hours, preferably from 1 second to 5 hours, more preferably from 1 minute to 1 hour.

Alternatively, and according to the preferred embodiment, the continuous flow process is a fixed bed continuous flow process or a reactive (catalytic) distillation process with preferably a heterogeneous hydrogenation catalyst. To initiate or regenerate the heterogeneous catalyst or to improve performance, fresh catalyst or catalyst regenerators may be added to the feed of the fixed bed or reactive distillation continuous flow process. In a fixed bed process, the liquid hourly space velocity (LHSV) can be from 1 to 1000, preferably from 5 to 500, more preferably from 10 to 250 and most preferably from 25 to 100.

Of particular interest is the combination of both aforementioned processes in a single process line, involving two (or more) reactor vessels in sequence or in two (or more) reaction zones of a fixed bed reactor. It will be appreciated that in this particular case, also water must be present to generate the hydrogen for the hydrogenation step. Alternatively, the reactions may also be carried out in batch, using a single reactor vessel. Various modifications of this process may be used without departing from the gist of this invention.

### EXAMPLES

The following examples are intended to further illustrate, without limiting, the processes of the invention.

### Example 1

A teflon lined, 10 mL stainless steel batch reactor containing 800 mg (5.2 mmol) of 5-(ethoxymethyl)furfural and 50.4 mg of a 5% Pd on active carbon catalyst (Degussa; E 1002 XU/W 5% Pd) is pressurized to 12.5 bar of hydrogen and subsequently heated, under stirring, to 200 °C for 3 hours. After the reaction, the reactor is cooled quickly in an ice bath and depressurized. A sample is diluted with methanol for analysis of the products with GC and GC-MS. The analysis shows a 5-(ethoxymethyl)furfural conversion of 34.1 % and a selectivity to ethoxymethylfuran of 36%. The main reason for the low selectivity is the competing aldehyde reduction reaction, giving 50% of 5-(ethoxymethyl)-2-hydroxymethylfuran, and further reduction to methylfurans, both resulting from catalyst activation by adding 12.5 bars of hydrogen.

### Example 2

A teflon lined, 10 mL stainless steel batch reactor containing 800 mg (5.2 mmol) of 5-(ethoxymethyl)furfural and 50.1 mg of a Ni on silica catalyst (KataLeuna; Supplier ID: KL6504N) is pressurized to 12.5 bar of hydrogen and subsequently heated, under stirring, to 200 °C for 3 hours. After the reaction, de reactor is cooled quickly in an ice bath and depressurized. A sample is diluted with methanol for analysis of the products with GC and GC-MS. The analysis shows a 5-(ethoxymethyl)furfural conversion of 41.4 % and a selectivity to ethoxymethylfuran of 25%. The main reason for the low selectivity is the competing aldehyde reduction reaction, giving 37% of 5-(ethoxymethyl)-2-hydroxymethylfuran, and 37% of further reduced melhylfurans, both resulting from catalyst activation by adding 12.5 bars of hydrogen.

### Example 3.

A teflon lined, 10 mL stainless steel batch reactor containing 160 mg (1.3 mmol) of 2-(ethoxymethyl)furan in 1 mL dioxane and 10.1 mg of a Ni on silica catalyst (KataLeuna; Supplier ID: KL6504N) is pressurized to 50 bar of hydrogen and subsequently heated, under stirring, to 120 °C for 3 hours. After the reaction, de reactor is cooled quickly in an ice bath and depressurized. A sample is diluted with methanol for analysis of the products with GC and GC-MS. The analysis shows a 2-(ethoxymethyl)furan conversion of 100 % and a selectivity to 2-(ethoxymethyl)tetrahydrofuran of 91%.

### Example 4.

Physical characteristics of the 2-(alkoxymethyl)tetrahydrofuran and bis-2,5-(alkoxymethyl)tetrahydrofuran compared to Jet A-1 aviation fuel and dimethylfuran (where alkoxymethyl = methoxymethyl, ethoxymethyl, *n*-propoxymethyl, *i*-propoxymethyl, *n-*butoxymethyl, *i*-butoxymethyl or *t*-butoxymethyl.

| | Jet A-1 | Dimethylfuran | New fuel components |
|---|---|---|---|
| Aromatics (%) | 25 | 100 ? | 0 - 100 |
| Flash point (°C) | 38 | -1.7 | Up to 60 |
| Melting point (°C) | - 47 | - 62 | -120 - -30 |
| Boiling point (°C) | range | 92 - 94 | 80 - >200 |
| Density (15°C, kg/m3) | 775-840 | 900 | 850 - 1100 |
| Net heat of combustion (MJ/L) | 33 | 33 | 28-34 |

### Example 5. fuel applications

### Fuel solubility

Fuel solubility is a primary concern for gasoline and diesel fuel applications. Not all highly polar oxygenates have good solubility in the current commercial gasoline and diesel fuels. Experiments show that the new fuel components according to the present invention can be blended with kerosene, gasoline and diesel in all ratios. In a comparative set of experiments it was shown that 5-(*t*-butoxymethyl)furfural can be blended with commercial diesel up to 45%. At higher blend concentration, phase separation was observed. 5-(ethoxymethyl)furfural (EMF) is miscible up to 5 vol% with commercial diesel.

### Cetane number

Oxygenated fuel additives may reduce the natural cetane number of the base diesel fuel. Blends of respectively 0.1 vol% of 5-(methoxymethyl)furan, 5-(methoxymethyl)tetrahydrofuran, 5-(ethoxymethyl)furan or 5-(ethoxymethyl)tetrahydrofuran were prepared with additive free diesel fuel at an outside laboratory for cetane determination according to an ASTM D 6890 certified method. While the reference additive-free diesel showed an average cetane number of 52.5, surprisingly, 0.1 vol% of the 5-(alkoxymethyl)furan and -tetrahydrofuran blends showed an increase with 0.5 - 1.0 to an average cetane number of 53.0 - 53.5.

### Oxidation stability

Likewise, oxygenated fuel additives often reduce the oxidation stability of the base diesel fuel. Blends of respectively 0.1 vol% of 5-(methoxymethyl)furan, 5-(methoxymethyl)tetrahydrofuran, 5-(ethoxymethyl)furan or 5-(ethoxymethyl)tetrahydrofuran were prepared with additive free diesel fuel at an outside laboratory for oxidation stability determination according to NF en ISO 12205 certified methods. Surprisingly, both the reference additive-free diesel and the four 0.1 vol% 5-(alkoxymethyl)furan and - tetrahydrofuran blends showed the same oxidation stability, indicating that the addition of new fuel components according to the present invention added an additive free diesel base fuel does not decrease the oxidation stability of the blend relative to the pure base diesel.

### Example 6. Diesel engine testing

In a D9B diesel engine of a citroen Berlingo test car, comparative testing is performed with normal commercial diesel as a fuel and the same commercial diesel to which 25 vol. % and 40 vol-% 5-(ethoxymethyl)furan was added, respectively 5-(ethoxymethyl)furan is added as a liquid and does not yield any mixing or flocculation problems at any blend ratio. The engine is run stationary with regular diesel initially, after which the fuel supply is switched to the 25 vol% 5-(ethoxymethyl)furan/diesel blend for 1.5 hours. After this time, the fuel supply is switched to the 40 vol% 5-(ethoxymethyl)furan/diesel blend for 1.5 hours.

## Claims

1. A process for preparing a monosubstituted furan derivative in the form of a 2-(alkoxymethyl)furan, for use as fuel component, by reacting a 5-(alkoxymethyl)furfural under decarbonylation conditions.

2. The process according to claim 1, wherein the reaction is carried out at a temperature from 50 to 300 degrees Celsius, preferably from 80 to 250 degrees Celsius, or more preferably from 150 to 225 degrees Celsius.

3. The process according to any one of the previous claims, wherein the catalyst is a heterogeneous catalyst.

4. The process according to any one of the previous claims, wherein the catalyst is a supported heterogeneous catalyst containing platinum, rhodium, ruthenium, palladium or nickel or mixture thereof (preferably in an amount of from 0,01 to 10 % by weight) and containing sodium, potassium and/or cesium metal (preferably in an amount from 0.1 to 10.0% by weight).

5. The process according to any one of the previous claims, wherein a heterogeneous catalyst is used, activated with hydrogen.

6. The process of any one of the previous claims, wherein a 5-(alkoxymethyl)furfural is converted into a 2-(alkoxymethyl)furan.

7. The process of claim 6, wherein the alkoxy group has from 1 to 20 carbon atoms, preferably from 1 to 12 carbon atoms and more preferably from 2 to 4 carbon atoms, optionally includes oxygen and/or nitrogen atoms in its chain or attached thereto, and may be linear or branched.

8. A process for preparing a monosubstituted tetrahydrofuran derivative in the form of a 2-(alkoxymethyl) tetrahydrofuran, for use as fuel component, wherein the monosubstituted furan derivative as prepared by the process of any one of claims 1 to 7 is converted under hydrogenation conditions into the tetrahydrofuran analog.

9. The process according to claim 8, wherein the reaction is carried out at a temperature from 50 to 400 degrees Celsius, preferably from 100 to 250, or more preferably from 100 to 150 degrees Celsius.

10. The process according to claim 9, wherein the reaction is carried out at a hydrogen pressure from 1 to 100 bar, or more preferably from 5 to 15 bar, where the molar ratio of hydrogen to furan derivative is not less than 5.

11. The process according to any one of claims 8 to 10, wherein the catalyst is a heterogeneous catalyst.

12. The process according to any one of the claims 8 to 11, wherein the catalyst is a supported metal heterogeneous hydrogenation catalyst containing base metals Ni, Cu, Co and Ca and/or precious metals Pt, Pd, Rh and Ru supported on alumina, titanium dioxide, silica, aluminium silicates, zeolites, magnesium silicate and active carbon or more preferably, the catalyst is a heterogeneous catalyst containing Ni, Pd or Rh supported on silica or carbon.

13. The process of any one of the claims 8 to 12, wherein a 2-(alkoxymethyl)furan is converted into a 2-(alkoxymethyl)tetrahydrofuran.

14. A monosubstituted furan derivative in the form of a 2-(C1-20 alkoxymethyl)furan as prepared by the process of any one of claims 1 to 7.

15. A monosubstituted tetrahydrofuran derivative in the form of a 2-(C1-20 alkoxymethyl)tetrahydrofuran as prepared by the process of any one of claims 8 to 13.

16. The use of the monosubstituted furan derivative of claim 14 as fuel component.

17. The use of the monosubstituted tetrahydrofuran derivative of claim 15 as fuel component.

18. The use of the monosubstituted tetrahydrofuran derivative of claim 17 as aviation fuel component.

19. Fuel comprising at least one of 2-(C1-20 alkoxymethyl)furan.

20. Fuel comprising at least one of 2-(C1-20 alkoxymethyl)tetrahydrofuran.

21. Aviation fuel comprising at least one of 2-(C1-20 alkoxymethyl)tetrahydrofuran.

22. Fuel comprising a combination of at least one of 2-(C1-20 alkoxymethyl)furan and at least one of 2-(C1-20 alkoxymethyl)tetrahydrofuran.

23. Aviation fuel comprising a combination of at least one of 2-(C1-20 alkoxymethyl)furan and at least one of 2-(C1-20 alkoxymethyl)tetrahydrofuran.
